# EUROPÄISCHE PATENTANMELDUNG

(11) **EP 4 148 742 A1**
(43) Veröffentlichungstag der Anmeldung: **15.03.2023**
(21) Anmeldenummer: 21196309.5
(22) Anmeldetag: 13.09.2021
(51) Int. Cl.: G16H 30/20, G16H 30/40, G16H 40/63, G16H 40/67

(54) **VORRICHTUNG UND VERFAHREN ZUR UNTERSTÜTZUNG EINER ÜBERWACHUNG EINES PATIENTEN**

(71) Anmelder: Siemens Healthcare GmbH, 91052 Erlangen (DE)
(72) Erfinder: Horn, Felix, 91154 Roth (DE); Nufer, Stephan, 91056 Erlangen (DE)

(57) **Zusammenfassung**

Die Erfindung betrifft ein Überwachungssystem (30) für eine Überwachung eines Patienten (15) in einem Untersuchungsraum (40) einer Bildgebungsvorrichtung (10), umfassend eine Schnittstelle (31), eine Recheneinheit (32) und eine Ausgabeeinheit (33), wobei die Schnittstelle (31) dazu ausgebildet ist, Prozessdaten bezüglich der Bildgebungsuntersuchung zu empfangen und an die 4 zu übertragen und wobei die Recheneinheit (32) dazu ausgebildet ist, die Prozessdaten zu verarbeiten und die Ausgabeeinheit (33) anzusteuern, eine Information bezüglich der Bildgebungsuntersuchung (36) in Abhängigkeit der Prozessdaten bereitzustellen, wobei das Überwachungssystem (30) dazu ausgebildet ist, eine visuelle Überwachung des Patienten (15) sowie der Information bezüglich der Bildgebungsuntersuchung (36) mittels der Ausgabeeinheit (33) zu ermöglichen. Die Erfindung betrifft ferner ein Verfahren zum Bereitstellen einer Information bezüglich einer Bildgebungsuntersuchung eines Patienten (15) mit einem Überwachungssystem (30).

## Beschreibung

Die Erfindung betrifft ein Überwachungssystem für eine Überwachung eines Patienten in einem Untersuchungsraum einer Bildgebungsvorrichtung. Die Erfindung betrifft ferner ein Verfahren zum Bereitstellen einer Information bezüglich einer Bildgebungsuntersuchung eines Patienten mit einem Überwachungssystem.

Bei einer Bildgebungsuntersuchung eines Patienten ist es typischerweise erforderlich, dass ein Nutzer einer Bildgebungsvorrichtung, wie z. B. ein Mitglied eines medizinischen Personals, den Patienten über eine gesamte Dauer der Bildgebungsuntersuchung visuell überwachen kann. Eine solche Überwachung kann beispielsweise erforderlich sein, um festzustellen, ob ein Patient Hilfe benötigt oder eine vorbestimmte Atembewegung in korrekter Weise vollzieht. Ein Umfeld der Bildgebungsvorrichtung, wie z. B. ein starkes Magnetfeld oder eine ionisierende Strahlung, machen es dabei erforderlich, dass der Nutzer die Bildgebungsvorrichtung von einem separaten Kontrollraum aus steuert, während der zu untersuchende Patient innerhalb der Bildgebungsvorrichtung in einem Untersuchungsraum verbleibt. Daraus resultiert eine räumliche Distanz bzw. Trennung zwischen dem Nutzer und dem Patienten, welche eine Interaktion des Nutzers mit dem Patienten erschwert und eine Vorbereitung und/oder Durchführung der Bildgebungsuntersuchung behindern kann.

Die Überwachung des Patienten durch den Nutzer erfolgt üblicherweise mittels eines speziellen Überwachungsfensters, welches in Abhängigkeit der verwendeten Bildgebungsvorrichtung verschiedenen Ansprüchen hinsichtlich der Interaktion mit (elektromagnetischer) Strahlung genügen muss. Hierzu gehören eine möglichst hohe Undurchlässigkeit für ionisierende Strahlung, aber auch Strahlung im Hochfrequenzbereich, wie sie bei Magnetresonanzvorrichtungen zum Einsatz kommt. Solche Überwachungsfenster sind üblicherweise mit signifikanten Kosten verbunden. Ferner ist aufgrund von baulichen Bedingungen oder einem Ablauf der Bildgebungsuntersuchung nicht immer gewährleistet, dass der Nutzer ein Geschehen im Untersuchungsraum in ausreichender Weise überwachen kann.

Es ist daher eine Aufgabe der Erfindung, eine Überwachung eines Patienten bei einer Bildgebungsuntersuchung zu verbessern.

Diese Aufgabe wird durch die Gegenstände der unabhängigen Patentansprüche erfindungsgemäß gelöst. Vorteilhafte Ausführungsformen und zweckmäßige Weiterbildungen sind Gegenstand der Unteransprüche.

Das erfindungsgemäße Überwachungssystem für eine Überwachung eines Patienten in einem Untersuchungsraum einer Bildgebungsvorrichtung, umfasst eine Schnittstelle, eine Recheneinheit und eine Ausgabeeinheit.

Eine Bildgebungsvorrichtung kann ein beliebiges medizinisches Gerät darstellen, welches dazu ausgebildet ist, Bilddaten einer Körperregion eines Patienten zu erfassen. Vorzugsweise ist eine Bildgebungsvorrichtung dazu ausgebildet, zweidimensionale und/oder dreidimensionale Bilddaten, insbesondere zeitabhängige dreidimensionale Bilddaten, der Körperregion des Patienten aufzunehmen. Beispiele für Bildgebungsvorrichtungen sind Magnetresonanzvorrichtungen, Röntgenvorrichtungen, Computertomografen, Einzelphotonen-Emissions-Computertomografen, Positronen-Emissions-Tomografen, aber auch Mammografievorrichtungen und dergleichen. In einer bevorzugten Ausführungsform ist die Bildgebungsvorrichtung als eine Magnetresonanzvorrichtung ausgestaltet.

Das Überwachungssystem weist Komponenten, wie z. B. die Schnittstelle, die Recheneinheit und die Ausgabeeinheit, auf. Darüber hinaus kann das Überwachungssystem weitere Komponenten, wie z. B. einen Sensor, eine Speichereinheit, eine Steuereinheit, Signalverbindungen zwischen den Komponenten und dergleichen, aufweisen. Die Komponenten des Überwachungssystems können elektrisch und/oder mechanisch miteinander verbunden sein. Es ist vorstellbar, dass die Komponenten eine strukturelle Einheit bilden oder an verschiedenen Orten in einem Untersuchungsraum und/oder einem Kontrollraum positioniert sind. Weiterhin können die Komponenten relativ zueinander positionierbar sein. Beispielsweise können die Ausgabeeinheit und die Recheneinheit in dem Kontrollraum positioniert sein, während die Schnittstelle in dem Untersuchungsraum positioniert ist. In einem weiteren Beispiel sind die Schnittstelle, die Recheneinheit und die Ausgabeeinheit in dem Kontrollraum positioniert, wobei die Schnittstelle mittels einer Signalverbindung mit einem Sensor und/oder der Bildgebungsvorrichtung in dem Untersuchungsraum verbunden ist. Eine Signalverbindung zwischen Komponenten in dem Kontrollraum und Komponenten in dem Untersuchungsraum ist vorzugsweise mittels einer faseroptischen Leitung bzw. einer optischen Verbindung ausgeführt. Es ist aber ebenso vorstellbar, dass die Signalverbindung mittels einer beliebigen elektrischen und/oder einer kabellosen Verbindung ausgeführt ist.

Die Schnittstelle ist dazu ausgebildet, Prozessdaten bezüglich der Bildgebungsuntersuchung zu empfangen und an die Recheneinheit zu übertragen. Es ist vorstellbar, dass die Schnittstelle als eine Kommunikationsschnittstelle ausgestaltet ist. Die Schnittstelle kann weiterhin als eine Datenschnittstelle und/oder als ein Sensor ausgebildet sein. Der Sensor ist vorzugsweise dazu ausgebildet, Prozessdaten bezüglich der Bildgebungsuntersuchung zu erfassen.

Prozessdaten können beliebige Daten darstellen, welche mit der Bildgebungsuntersuchung zusammenhängen. Beispielsweise können die Prozessdaten physiologische Daten des Patienten, Patienteninformationen, Parameter der Bildgebungsuntersuchung, optische Bilddaten des Patienten oder dergleichen umfassen. Die Prozessdaten können mittels der Schnittstelle von einem Sensor, der Bildgebungsvorrichtung und/oder einer beliebigen bildgebungsrelevanten Komponente empfangen werden.

Die Recheneinheit ist dazu ausgebildet, die Prozessdaten zu verarbeiten und die Ausgabeeinheit anzusteuern, eine Information bezüglich der Bildgebungsuntersuchung in Abhängigkeit der Prozessdaten bereitzustellen. Die Ausgabeeinheit kann ein beliebiges Ausgabegerät, wie z. B einen Bildschirm, einen transparenten Bildschirm, einen Projektor, einen Touchscreen, einen Lautsprecher, aber auch eine Mehrzahl solcher Ausgabegeräte umfassen. Die Recheneinheit weist vorzugsweise eine CPU (central processing unit) und/oder eine GPU (graphics processing unit) auf, welche eine Verarbeitung von Prozessdaten, insbesondere auch optischen Daten und/oder Bilddaten, ermöglichen. Das Verarbeiten der Prozessdaten kann insbesondere ein Extrahieren von Informationen, ein Korrelieren von Informationen, ein Komprimieren von Daten, ein Umwandeln bzw. Konvertieren von Daten, ein Klassifizieren von Daten und/oder ein Auswählen von Daten umfassen.

Ein Nutzer der Bildgebungsvorrichtung kann insbesondere ein Mitglied eines medizinischen Personals, wie z. B. ein Pfleger, eine Ärztin, ein medizinisch-technischer Radiologie-Assistent (MTRA) oder dergleichen, darstellen.

Eine Bildgebungsuntersuchung kann eine bildgebende Untersuchung eines Patienten zur Erfassung von Bildern einer diagnostisch relevanten Körperregion des Patienten darstellen. Eine Bildgebungsuntersuchung kann insbesondere einen Vorgang darstellen, bei welchem hochfrequente elektromagnetische Wellen in eine Bildaufnahmeregion der Bildgebungsvorrichtung ausgesendet und hochfrequente Signale mit einem Frequenz- und Leistungsbereich üblicher Magnetresonanzsignale aus der Bildaufnahmeregion erfasst werden

Die Bildgebungsuntersuchung umfasst vorzugsweise alle zur Erfassung von diagnostischen Bilddaten der Körperregion des Patienten erforderlichen Vorgänge und/oder Phasen, insbesondere eine Planung, eine Vorbereitung und eine Durchführung der Bildgebungsuntersuchung. Die Bildgebungsuntersuchung kann einen oder mehrere Parameter, insbesondere Bildgebungsparameter, und/oder eine oder mehrere Bildgebungssequenzen umfassen. Ein Bildgebungsparameter kann eine Randbedingung oder eine Variable einer Bildgebungssequenz darstellen, mit welcher sich das Erfassen von diagnostischen Bilddaten der Körperregion des Patienten einstellen lässt.

Die Bildgebungsuntersuchung kann ferner einen oder mehrere Vorgänge umfassen. Ein Vorgang kann einen beliebigen Abschnitt der Bildgebungsuntersuchung darstellen. Beispielsweise kann ein Vorgang eine Positionierung eines Patiententischs, eine Positionierung eines Patienten, eine Positionierung einer Körperregion des Patienten, eine Positionierung einer Lokalspule, ein Starten einer Bildgebungssequenz, eine Registrierung des Patienten, ein Aufklären des Patienten, eine Anästhesie, einen chirurgischen Eingriff, einen minimalinvasiven Eingriff oder dergleichen umfassen.

Eine Information bezüglich der Bildgebungsuntersuchung kann eine beliebige Information betreffen, welche für die Überwachung des Patienten und/oder eine Koordination der Bildgebungsuntersuchung relevant ist. Die Information bezüglich der Bildgebungsuntersuchung kann beliebige, in Abhängigkeit der Prozessdaten ermittelte, Informationen umfassen. Vorzugsweise umfasst die Information bezüglich der Bildgebungsuntersuchung eine Information über einen physiologischen Zustand und/oder eine Befindlichkeit des Patienten.

Die Information bezüglich der Bildgebungsuntersuchung kann mittels einer Korrelation von Prozessdaten erhalten werden. Es ist weiterhin vorstellbar, dass die Information bezüglich der Bildgebungsuntersuchung Bilder bzw. bewegte Bilder umfasst, welche auf Basis der Prozessdaten erstellt werden. Die Bilder bzw. bewegten Bilder können dabei insbesondere den Patienten in dem Untersuchungsraum betreffen. In einem Beispiel ist die Recheneinheit dazu ausgebildet, mittels eines optischen Sensors, wie z. B. eines CCD-Sensors und/oder eines CMOS, erfasste optische Bilddaten in Bilder bzw. bewegte Bilder umzuwandeln und mittels der Ausgabeeinheit bereitzustellen. In einem weiteren Beispiel ist die Recheneinheit dazu ausgebildet, die Information bezüglich der Bildgebungsuntersuchung anhand von Bilddaten des optischen Sensors zu extrahieren. Eine solche Information bezüglich der Bildgebungsuntersuchung kann beispielsweise eine Atemkurve sein, welche in Abhängigkeit einer Videoaufnahme des Patienten ermittelt wird.

Die Information bezüglich der Bildgebungsuntersuchung kann weiterhin eine von einem Vorgang der Bildgebungsuntersuchung abhängige Handlung des Nutzers definieren und/oder auslösen. Beispiele hierfür sind eine Positionierung des Patienten und/oder einer bildgebungsrelevanten Komponente für die Bildgebungsuntersuchung, einen Abbruch und/oder eine Wiederholung eines Abschnitts der Bildgebungsuntersuchung, aber auch eine Darstellung ausstehender und/oder abgeschlossener Schritte der Bildgebungsuntersuchung.

Das Überwachungssystem ist dazu ausgebildet, eine visuelle Überwachung des Patienten sowie der Information bezüglich der Bildgebungsuntersuchung mittels der Ausgabeeinheit zu ermöglichen. Vorzugsweise ist die Ausgabeeinheit dazu ausgebildet, die Information bezüglich der Bildgebungsuntersuchung einem Sichtbereich auf den Patienten und/oder einem digitalen Abbild des Patienten zu überlagern. Dies kann bedeuten, dass die Information bezüglich der Bildgebungsuntersuchung in dem Sichtbereich des Nutzers positioniert ist, während der Nutzer den Patienten visuell überwacht. Ein Verdecken eines Umrisses oder einer Kontur des Patienten durch die Information bezüglich der Bildgebungsuntersuchung kann dabei vermieden werden. Das Überwachungssystem ist insbesondere dazu ausgebildet, die visuelle Überwachung des Patienten sowie die Information bezüglich der Bildgebungsuntersuchung parallel bzw. zeitgleich an den Nutzer der Bildgebungsvorrichtung auszugeben.

Mittels des erfindungsgemäßen Überwachungssystems lassen sich Informationen bezüglich der Bildgebungsuntersuchung in den Sichtbereich des Nutzers auf den Patienten integrieren bzw. diesem überlagern. Dadurch kann eine Ablenkung des Nutzers, z. B. aufgrund eines Aufrufens von Patienteninformationen und/oder einer Überwachung von Parametern der Bildgebungsuntersuchung mittels eines separaten Systems, auf vorteilhafte Weise reduziert oder vermieden werden. Somit lassen sich eine Effizienz und/oder eine Qualität der Überwachung des Patienten vorteilhaft erhöhen.

In einer Ausführungsform des erfindungsgemäßen Überwachungssystems umfasst die Ausgabeeinheit einen Projektor, wobei der Projektor dazu ausgebildet ist, die Information bezüglich der Bildgebungsuntersuchung auf ein Überwachungsfenster des Untersuchungsraums zu projizieren. Der Projektor kann dabei in dem Untersuchungsraum oder dem Kontrollraum positioniert sein. Vorzugsweise ist der Projektor dazu ausgebildet, die Information bezüglich der Bildgebungsuntersuchung in Form von Bildern bzw. graphischen Repräsentationen auf das Überwachungsfenster zu projizieren.

Das Überwachungsfenster ist dazu ausgebildet, einem Nutzer der Bildgebungsvorrichtung eine Sicht auf den Patienten in dem Untersuchungsraum zu ermöglichen, wobei die Recheneinheit dazu ausgebildet ist, die Ausgabeeinheit anzusteuern, die Information bezüglich der Bildgebungsuntersuchung mittels des Projektors einem Sichtbereich des Nutzers durch das Überwachungsfenster zu überlagern. Die Recheneinheit ist vorzugsweise dazu ausgebildet, die Information bezüglich der Bildgebungsuntersuchung derart bereitzustellen, dass die Information bezüglich der Bildgebungsvorrichtung einer Anzeige bzw. einem Bild des Patienten und/oder einem Sichtbereich des Nutzers auf den Patienten, überlagert ist.

Das Überwachungsfenster kann ein Teil des Überwachungssystems sein oder separat von dem Überwachungssystem vorliegen.

Vorzugsweise ist das Überwachungsfenster transparent, um eine Sicht aus dem Kontrollraum auf den Patienten im Untersuchungsraum zu ermöglichen. Das Überwachungsfenster kann auf einer dem Projektor zugewandten Seite eine Schicht bzw. eine Beschichtung aufweisen, welche dazu ausgebildet ist, ein optisches Signal des Projektors derart zu brechen, dass eine Sichtbarkeit der Information bezüglich der Bildgebungsuntersuchung für den Nutzer ermöglicht oder verbessert wird.

Ein Projektor stellt eine besonders kostengünstige und/oder einfache zu implementierende Möglichkeit dar, die Information bezüglich der Bildgebungsuntersuchung dem Sichtbereich des Nutzers zu überlagern, ohne die visuelle Überwachung des Patienten durch den Nutzer zu kompromittieren.

In einer weiteren Ausführungsform des erfindungsgemäßen Überwachungssystems weist die Ausgabeeinheit einen transparenten Bildschirm auf, welcher dazu ausgebildet ist, einem Nutzer der Bildgebungsvorrichtung eine Sicht in den Untersuchungsraum durch den transparenten Bildschirm zu ermöglichen. Bei einem transparenten Bildschirm kann eine aktive Matrix bzw. ein Bildbereich des Bildschirms transparent gestaltet sein, sodass ein Nutzer durch den Bildschirm hindurchblicken kann. Die aktive Matrix oder der Bildbereich kann dabei durch eine Anordnung von individuellen Bildelementen (Pixel) mit elektronischen Bauelementen, wie z. B. einer Diode, einem Transistor, einem Kondensator oder dergleichen, charakterisiert sein. Die individuellen Bildelemente können in ein transparentes Substrat eingebettet sein oder von einem solchen gehalten werden. Eine Technologie des transparenten Bildschirms beruht vorzugsweise auf einer LCD, einer LED oder einer OLED Technologie.

Die Recheneinheit ist dazu ausgebildet, die Ausgabeeinheit anzusteuern, die Information bezüglich der Bildgebungsuntersuchung mittels des transparenten Bildschirms einem Sichtbereich des Nutzers durch den transparenten Bildschirm zu überlagern. Die Recheneinheit kann ferner dazu ausgebildet sein, die Information bezüglich der Bildgebungsuntersuchung derart bereitzustellen, dass die Information bezüglich der Bildgebungsvorrichtung einer Anzeige des Patienten und/oder einem Sichtbereich des Nutzers auf den Patienten durch den transparenten Bildschirm, überlagert ist. Vorzugsweise ist die Recheneinheit weiterhin dazu ausgebildet, Informationen bezüglich der Bildgebungsuntersuchung, welche den Patienten und/oder eine bildgebungsrelevante Komponente betreffen, derart bereitzustellen, dass die Informationen bezüglich der Bildgebungsuntersuchung in dem Sichtfeld des Nutzers an dem Patienten und/oder der bildgebungsrelevanten Komponente angeordnet sind.

Durch das Bereitstellen des transparenten Bildschirms lassen sich beliebige Informationen bezüglich der Bildgebungsuntersuchung auf vorteilhafte Weise in dem Sichtfeld des Nutzers anordnen, sodass die Überwachung des Patienten in dem Untersuchungsraum verbessert werden kann. Ferner können auf dem transparenten Bildschirm auch mittels einer Kamera erfasste Bilder des Patienten angeordnet werden, sodass visuelle Überwachungsmöglichkeiten des Nutzers vorteilhaft erweitert werden. Weiterhin kann bei Verwendung eines Projektors und/oder eines transparenten Bildschirms auch dann noch eine visuelle Überwachung des Patienten ermöglicht werden, wenn ein Bereitstellen der Information bezüglich der Bildgebungsuntersuchung aufgrund eines Ausfalls einer Komponente des Überwachungssystems unmöglich ist.

Gemäß einer weiteren Ausführungsform weist das erfindungsgemäße Überwachungssystem eine Kamera auf, welche in dem Untersuchungsraum positioniert ist. Die Kamera ist dazu ausgebildet, optische Bilddaten des Patienten in dem Untersuchungsraum zu erfassen und an die Schnittstelle zu übertragen, wobei die Recheneinheit dazu ausgebildet ist, Bilder des Patienten in Abhängigkeit der optischen Bilddaten zu erstellen und mittels der Ausgabeeinheit bereitzustellen.

Die Kamera kann beispielsweise als eine 2D Kamera, eine 3D Kamera und/oder eine Infrarotkamera ausgestaltet sein. Vorzugsweise weist das Überwachungssystem eine Mehrzahl von Kameras auf, welche optische Bilddaten den Patienten aus verschiedenen Richtungen erfassen. In einer Ausführungsform weist das Überwachungssystem eine erste Kamera auf, welche dazu ausgebildet ist, optische Bilddaten eines Abschnitts des Patienten zu erfassen, welcher außerhalb einer Bildgebungsregion der Bildgebungsvorrichtung auf einer Patientenlagerungsvorrichtung der Bildgebungsvorrichtung positioniert ist. Das Überwachungssystem kann ferner eine zweite Kamera aufweisen, welche dazu ausgebildet ist, optische Bilddaten eines Abschnitts des Patienten zu erfassen, welcher innerhalb der Bildgebungsregion positioniert ist. In einer Ausführungsform ist die Recheneinheit des Überwachungssystems dazu ausgebildet, eine Position des Patienten in dem Untersuchungsraum, insbesondere eine relative Position des Patienten zu der Bildgebungsvorrichtung, zu ermitteln. In Abhängigkeit der ermittelten Position des Patienten kann die Recheneinheit die Information bezüglicher der Bildgebungsuntersuchung anpassen. Beispielsweise kann die Recheneinheit dazu ausgebildet sein, optische Bilddaten einer oder mehrerer Kameras in Abhängigkeit der relativen Position des Patienten zu der Bildgebungsvorrichtung auszuwählen und dem Nutzer mittels der Ausgabeeinheit automatisch als Bild und/oder Video bereitzustellen. Die Recheneinheit kann ebenso dazu ausgebildet sein, einen von der ersten Kamera und/oder der zweiten Kamera erfassten Gesichtsausdruck des Patienten zu ermitteln und/oder zu bewerten, um die Information über den physiologischen Zustand und/oder die Befindlichkeit des Patienten zu bestimmen.

Das Überwachungssystem kann ferner zumindest einen Sensor aufweisen, welcher dazu ausgebildet ist, die relative Position des Patienten zu der Bildgebungsvorrichtung und/oder eine Position einer bildgebungsrelevanten Komponente, zu ermitteln. Ein solcher Sensor kann beispielsweise ein Abstandssensor und/oder ein Positionsgeber an einer bildgebungsrelevanten Komponente, wie z. B. der Patientenlagerungsvorrichtung, sein. Gleichermaßen kann eine Kamera als ein solcher Sensor verstanden werden. In diesem Fall weist die Recheneinheit vorzugsweise eine Bildverarbeitungseinheit auf, welche dazu ausgebildet ist, die relative Position des Patienten zu der Bildgebungsvorrichtung, aber auch eine Position des Patienten in dem Untersuchungsraum, zu ermitteln. Neben dem Untersuchungsraum, kann eine Kamera auch in einem Patienten-Wartebereich positioniert sein, um optische Bilddaten von Patienten in dem Patienten-Wartebereich zu erfassen.

Durch das Bereitstellen einer Kamera oder einer Mehrzahl von Kameras lässt sich die Sicht des Nutzers auf den Patienten im Vergleich zu einem Überwachungsfenster, welches üblicherweise in einem größeren Abstand zu der Bildgebungsvorrichtung positioniert ist und einen vorbestimmten Sichtbereich aufweist, verbessern. Durch das Bereitstellen einer Mehrzahl von Kameras lassen sich optische Bilddaten des Patienten vorteilhaft aus verschiedenen Richtungen erfassen, wodurch die Überwachung des Patienten weiter verbessert werden kann. Ferner kann durch ein automatisches Auswählen einer Kamera, welche zu einem gegebenen Zeitpunkt eine gute oder optimale Sicht auf den Patienten ermöglicht, eine besonders vorteilhafte Überwachung des Patienten durch den Nutzer ermöglicht werden. Ein weiterer Vorteil besteht darin, dass die erfassten optischen Bilddaten des Patienten mittels der Ausgabeeinheit in einer beliebigen Größe mittels der Ausgabeeinheit bereitgestellt werden können. Dadurch können auch Mitglieder des medizinischen Personals, welche in größerer Distanz zu der Ausgabeeinheit des Überwachungssystems in dem Kontrollraum und/oder dem Untersuchungsraum positioniert sind, Informationen bezüglich der Bildgebungsuntersuchung und/oder Bilder des Patienten wahrnehmen.

In einer weiteren Ausführungsform des erfindungsgemäßen Überwachungssystems umfasst die Ausgabeeinheit einen Bildschirm. Ein Bildschirm kann ein beliebiger Bildschirm sein, welcher dazu ausgebildet ist, Bilder des Patienten anzuzeigen. Die Ausgabeeinheit kann insbesondere dazu ausgebildet ein, eine Bildwiederholungsrate von mehr als 20 Bilder pro Sekunde zu ermöglichen, um bewegte Bilder, beispielsweise ein Videosignal mit Bilddaten des Patienten, bereitzustellen. Eine aktive Matrix bzw. ein Bildbereich des Bildschirms ist bei dieser Ausführungsform vorzugsweise opak.

Die Recheneinheit ist dazu ausgebildet, Bilder des Patienten in dem Untersuchungsraum mittels des Bildschirms bereitzustellen. Die Recheneinheit kann insbesondere dazu ausgebildet sein, Bilder des Patienten mit einer weiteren Information bezüglich der Bildgebungsuntersuchung zu überlagern. Die Bilder des Patienten beruhen vorzugsweise auf optischen Bilddaten des Patienten, welche mittels eines Sensors, wie z. B. einer Kamera oder einer Mehrzahl von Kameras, erfasst werden. Es ist vorstellbar, dass die Recheneinheit dazu ausgebildet ist, die bereitzustellenden optischen Bilddaten des Patienten auszuwählen und mit einer weiteren Information bezüglich der Bildgebungsuntersuchung auf dem Bildbereich des Bildschirms zu überlagern. Die Recheneinheit kann ferner dazu ausgebildet sein, optische Bilddaten mehrerer Kameras zeitsnychron mittels des Bildschirms bereitzustellen.

Durch die Verwendung eines Bildschirms zur Überwachung des Patienten lassen sich Kosten für das Überwachungssystem im Vergleich zu einem konventionellen Überwachungsfensters auf vorteilhafte Weise reduzieren. Ferner kann durch eine automatische Auswahl optischer Bilddaten einer Mehrzahl von Kameras stets eine gute oder optimierte Sicht des Nutzers auf den Patienten gewährleistet werden.

In einer weiteren Ausführungsform weist das erfindungsgemäße Überwachungssystem zumindest einen Sensor auf, welcher in dem Untersuchungsraum der Bildgebungsvorrichtung positioniert ist und dazu ausgebildet ist, Prozessdaten aus dem Untersuchungsraum zu erfassen. Der zumindest eine Sensor kann dazu ausgebildet sein, beliebige Messdaten aus dem Untersuchungsraum zu erfassen. Beispielsweise kann der zumindest eine Sensor als ein Temperatursensor, ein Luftfeuchtigkeitssensor, ein Schallsensor, ein optischer Sensor, ein Sensor eines medizinischen Geräts oder dergleichen ausgestaltet sein. Der zumindest eine Sensor kann insbesondere als eine Kamera gemäß einer oben beschriebenen Ausführungsform ausgeführt sein. Es ist weiterhin vorstellbar, dass der zumindest eine Sensor als ein Positionsgeber und/oder ein Abstandsmesser ausgestaltet ist. Ein solcher Sensor kann insbesondere dazu ausgebildet sein, eine Position einer bildgebungsrelevanten Komponente, insbesondere einer Patientenlagerungsvorrichtung oder eines Patiententischs, zu erfassen. Die Recheneinheit kann entsprechend dazu ausgebildet sein, eine Kamera und/oder ein Signal einer Kamera auszuwählen und dem Nutzer mittels der Ausgabeeinheit bereitzustellen.

Die Schnittstelle ist dazu ausgebildet, die Prozessdaten von dem zumindest einen Sensor zu empfangen und an die Recheneinheit zu übertragen, wobei die Information bezüglich der Bildgebungsuntersuchung auf den Prozessdaten des zumindest einen Sensors beruht. Die Information bezüglich der Bildgebungsuntersuchung kann dabei Bilder und/oder bewegte Bilder umfassen, welche mittels der Recheneinheit aus erfassten optischen Bilddaten einer Kamera erstellt werden. Es ist ebenso vorstellbar, dass die Information bezüglich der Bildgebungsuntersuchung eine Markierung des Patienten, insbesondere einer Körperregion des Patienten, umfasst, welche mittels der Recheneinheit einem Sichtbereich des Nutzers bei der visuellen Überwachung des Patienten überlagert ist. Ferner kann die Information bezüglich der Bildgebungsuntersuchung eine grafische Repräsentation der Prozessdaten und/oder einem Ergebnis einer Korrelation mehrerer Prozessdaten umfassen, welche dem Sichtbereich des Nutzers bei der Überwachung des Patienten überlagert werden.

Durch das Bereitstellen zumindest eines Sensors, welcher dazu ausgebildet ist, Prozessdaten aus dem Untersuchungsraum zu erfassen, lässt sich eine Mehrzahl von für die Bildgebungsuntersuchung relevante Messdaten auf vorteilhafte Weise in einem Sichtbereich des Nutzers positionieren. Weiterhin lassen sich in Abhängigkeit von Prozessdaten des zumindest einen Sensors oder der Mehrzahl von Sensoren auf vorteilhafte Informationen generieren, welche für den Nutzer ansonsten unzugänglich oder nur unter Verwendung einer weiteren technischen Einrichtung nachvollziehbar wären. Somit kann eine konsistente und/oder ungestörte Überwachung des Patienten ermöglicht werden.

In einer weiteren Ausführungsform der erfindungsgemäßen Überwachungssystems ist der zumindest eine Sensor dazu ausgebildet, zumindest eines der folgenden Prozessdaten zu erfassen:
- einen medizinischen Parameter des Patienten,
- einen Parameter einer bildgebungsrelevanten Komponente,
- einen Messwert eines Medizingeräts.

Ein medizinischer Paramater des Patienten kann insbesondere Vitaldaten und/oder physiologische Messdaten des Patienten betreffen. Beispiele hierfür sind ein Atemverhalten, ein Elektrokardiogramm, eine Blut-Sauerstoffsättigung, eine Temperatur oder dergleichen. Ein Parameter einer bildgebungsrelevanten Komponente kann z. B. Positionsdaten eines Patiententischs oder einer Patientenlagerungsvorrichtung umfassen. Ein Messwert eines Medizingeräts kann beispielsweise Bilddaten einer weiteren Bildgebungsuntersuchung, wie z. B. einer Ultraschallmessung, einer Positronen-Emissionstomografiemessung, einer Einzelphotonen-Computertomografiemessung, aber auch einer Elastizitätsmessung oder dergleichen umfassen. Die in Abhängigkeit der Prozessdaten ermittelte Information bezüglich der Bildgebungsuntersuchung kann dabei einen Zustand des Patienten während der Bildgebungsuntersuchung betreffen. Es ist insbesondere vorstellbar, dass die Information bezüglich der Bildgebungsuntersuchung einen Warnhinweis oder einen Alarm umfasst, welcher auf einen kritischen Zustand eines Patienten hinweist. Ein solcher Hinweis kann aber einen Vorgang der Bildgebungsuntersuchung betreffen, bei welchem eine besondere Aufmerksamkeit des Nutzers gefordert ist.

Weiterhin kann der Hinweis auch Zustand der Bildgebungsvorrichtung, insbesondere einen Gerätestatus der Bildgebungsvorrichtung betreffen.

Anhand von physiologischen Messdaten des Patienten lässt sich eine visuelle Überwachung des Patienten um relevante Daten bezüglich des Zustands des Patienten erweitern. Dadurch kann eine Überwachung des Patienten während der Bildgebungsuntersuchung, insbesondere eine Identifikation einer negativen Reaktion des Patienten auf die Bildgebungsuntersuchung, auf vorteilhafte Weise verbessert werden. Durch das Bereitstellen weiterer, oben genannter Prozessdaten, kann dem Nutzer auf vorteilhafte Weise ein konsolidierter Überblick über einen Status des Patienten während der Bildgebungsuntersuchung gegeben werden.

Gemäß einer Ausführungsform des erfindungsgemäßen Überwachungssystems weist die Schnittstelle eine Signalverbindung mit der Bildgebungsvorrichtung auf. Die Schnittstelle ist dazu ausgebildet, Prozessdaten von der Bildgebungsvorrichtung zu empfangen und and die Recheneinheit zu übertragen, wobei die Prozessdaten einen Parameter der Bildgebungsuntersuchung und/oder eine Patienteninformation betreffen.

Ein Parameter der Bildgebungsuntersuchung kann ein beliebiger Parameter sein, welcher für die Bildgebungsuntersuchung und/oder einen Vorgang der Bildgebungsuntersuchung charakteristisch und/oder bestimmend ist. Ein Parameter der Bildgebungsuntersuchung kann insbesondere eine Bildgebungssequenz, eine Abfolge von Bildgebungssequenzen und/oder einen Bildgebungsparameter einer Bildgebungssequenz darstellen. Ein Bildgebungsparameter kann eine Randbedingung oder eine Variable einer Bildgebungssequenz umfassen, mit welcher sich ein Erfassen von Bilddaten einer Körperregion des Patienten einstellen lässt. Beispiele für Bildgebungsparameter sind eine Bildauflösung, ein Kontrast, ein Signal-zu-Rausch Verhältnis, eine spezifische Absorptionsrate, eine Echozeit, eine Repetitionszeit oder dergleichen. Es ist ebenso vorstellbar, dass der Parameter der Bildgebungsvorrichtung eine Gruppe von Bildgebungsparametern und/oder eine Abfolge von Bildgebungssequenzen umfasst. Weiterhin kann ein Parameter eine beliebige Einstellung der Bildgebungsuntersuchung und/oder eines Ablaufs der Bildgebungsuntersuchung umfassen.

Die Bildgebungsuntersuchung kann ferner einen oder mehrere Vorgänge umfassen. Ein Vorgang kann einen beliebigen Abschnitt der Bildgebungsuntersuchung darstellen. Beispielsweise kann ein Vorgang eine Positionierung eines Patiententischs, eine Positionierung eines Patienten, eine Positionierung einer Körperregion des Patienten, eine Positionierung einer Lokalspule, ein Starten einer Bildgebungssequenz, eine Registrierung des Patienten, eine Aufklärung des Patienten, eine Anästhesie, einen chirurgischen Eingriff, einen minimalinvasiven Eingriff oder dergleichen umfassen.

In einer bevorzugten Ausführungsform umfasst die Information bezüglich der Bildgebungsuntersuchung einen Fortschritt und/oder einen Status, eine verbleibende Dauer, eine aktuelle Bildgebungssequenz und/oder eine Information über einen Parameter bzw. einen Parametersatz der Bildgebungsuntersuchung. Die Recheneinheit ist entsprechend dazu ausgebildet, die Information bezüglich der Bildgebungsuntersuchung mittels der Ausgabeeinheit einem Sichtbereich des Nutzers bei der visuellen Überwachung des Patienten zu überlagern.

Eine Patienteninformation kann beliebige, patientenbezogenen Daten umfassen. Beispielsweise umfasst die Patienteninformation Registrierungsdaten des Patienten, aber auch Informationen über eine Krankenhistorie, ein Alter, ein Gewicht, ein Geschlecht und/oder demographische Daten des Patienten.

Vorzugsweise weist die Schnittstelle eine Signalverbindung mit einer Recheneinheit und/oder einer Steuereinheit der Bildgebungsvorrichtung auf, um die Prozessdaten von der Bildgebungsvorrichtung zu empfangen. Die Signalverbindung kann dabei kabellos oder kabelgebunden gemäß einer oben beschriebenen Ausführungsform ausgestaltet sein.

Durch ein Bereitstellen einer Patienteninformation in einem Sichtbereich des Nutzers auf den Patienten, kann der Nutzer auf vorteilhafte Weise auf einen Zustand und/oder eine Vorerkrankung des Patienten hingewiesen werden, welche eine Durchführung der Bildgebungsuntersuchung erschweren können. Mittels des Bereitstellens der Information bezüglich der Bildgebungsuntersuchung auf Basis eines Parameters der Bildgebungsuntersuchung kann der Nutzer vorteilhaft über einen Ablauf und/oder einen Fortschritt der Bildgebungsuntersuchung hingewiesen werden, ohne die visuelle Überwachung des Patienten zu unterbrechen.

In einer bevorzugten Ausführungsform des erfindungsgemäßen Überwachungssystems ist die Recheneinheit dazu ausgebildet, eine Mehrzahl von Informationen bezüglich der Bildgebungsuntersuchung mittels der Ausgabeeinheit bereitzustellen. Die Mehrzahl der Informationen bezüglich der Bildgebungsuntersuchung kann dabei insbesondere auf einer Mehrzahl von Prozessdaten gemäß der oben beschriebenen Ausführungsformen beruhen.

Die Recheneinheit ist weiterhin dazu ausgebildet, eine Art, eine Anordnung und/oder einen Zeitpunkt der bereitgestellten Informationen bezüglich der Bildgebungsuntersuchung in Abhängigkeit der Prozessdaten anzupassen. Die Recheneinheit kann insbesondere dazu ausgebildet sein, die Informationen bezüglich der Bildgebungsuntersuchung derart mittels der Ausgabeeinheit anzuordnen, dass eine direkte Sicht auf den Patienten uneingeschränkt möglich ist. Beispielsweise kann die Recheneinheit dazu ausgebildet sein, die Informationen bezüglich der Bildgebungsuntersuchung mittels der Ausgabeeinheit derart in dem Sichtbereich des Nutzers anzuordnen, dass ein Umriss oder eine Kontur des Patienten von den bereitgestellten Informationen bezüglich der Bildgebungsuntersuchung unverdeckt bleibt. Es ist ferner vorstellbar, dass Informationen bezüglich der Bildgebungsuntersuchung mit Bezug zu Vitaldaten des Patienten in dem Sichtbereich des Nutzers in einer unmittelbaren Nähe zu dem Patienten angeordnet sind. Solche Informationen bezüglich der Bildgebungsuntersuchung können den Umriss bzw. die Kontur des Patienten in dem Sichtbereich des Nutzers teilweise oder vollständig überlagern oder ein Verdecken des Patienten vermeiden.

In einer Ausführungsform ist die Recheneinheit dazu ausgebildet, die Art, Anordnung und/oder den Zeitpunkt der bereitgestellten Informationen in Abhängigkeit einer Position des Nutzers in dem Kontrollraum und/oder einer Richtung einer Sicht des Nutzers auf den Patienten anzupassen. Es ist ferner vorstellbar, dass ausgewählte Informationen bezüglich der Bildgebungsuntersuchung, wie z. B. eine verbleibende Dauer der Bildgebungsuntersuchung oder ein Wechsel einer Bildgebungssequenz, zu vorbestimmten Zeitpunkten an den Nutzer ausgegeben werden. Ein vorbestimmter Zeitraum kann z. B. ein anstehendes Ende der Bildgebungsuntersuchung und/oder ein Wechsel einer Bildgebungssequenz sein. Weiterhin kann die Recheneinheit dazu ausgebildet sein, Informationen bezüglich der Bildgebungsuntersuchung mit aktueller Relevanz für die Überwachung des Patienten und/oder die Durchführung der Bildgebungsuntersuchung hervorzuheben. Das Hervorheben kann beispielsweise eine Auswahl, aber auch eine Anpassung einer Farbe und/oder einer Größe der Information bezüglich der Bildgebungsuntersuchung sowie eine Markierung entsprechender Informationen bezüglich der Bildgebungsuntersuchung umfassen.

Durch das Anpassen der Art, Anordnung und/oder des Zeitpunkts der bereitgestellten Informationen bezüglich der Bildgebungsuntersuchung kann eine Sicht des Nutzers auf den Patienten auf vorteilhafte Weise verbessert oder optimiert werden. Ferner lassen sich die Informationen bezüglich der Bildgebungsuntersuchung vorteilhaft in Abhängigkeit ihrer Relevanz dem Nutzer bereitstellen, wodurch eine Überlastung oder Ablenkung des Nutzers durch eine Vielzahl von Informationen bezüglich der Bildgebungsuntersuchung vermieden werden kann.

Das erfindungsgemäße Verfahren zum Bereitstellen einer Information bezüglich einer Bildgebungsuntersuchung eines Patienten mit einem Überwachungssystem mit einer Schnittstelle, einer Recheneinheit und einer Ausgabeeinheit weist die folgenden Schritte auf:
- Empfangen von Prozessdaten mittels der Schnittstelle,
- Verarbeiten der Prozessdaten mittels der Recheneinheit und
- Bereitstellen der Information bezüglich der Bildgebungsuntersuchung in Abhängigkeit der Prozessdaten mittels der Ausgabeeinheit.

Das Überwachungssystem kann gemäß einer oben beschriebenen Ausführungsform ausgestaltet sein. Das erfindungsgemäße Verfahren teilt die Vorteile des erfindungsgemäßen Überwachungssystems.

In einer Ausführungsform des erfindungsgemäßen Verfahrens umfasst das Empfangen von Prozessdaten ein Empfangen von optischen Bilddaten einer Mehrzahl von Kameras. Vorzugsweise erfasst die Mehrzahl von Kameras optische Bilddaten des Patienten aus verschiedenen Richtungen. Die Mehrzahl von Kameras kann dabei eine erste Kamera und eine zweite Kamera umfassen. Die erste Kamera kann optische Bilddaten des Patienten auf der Patientenlagerungsvorrichtung aus einer Vogelperspektive erfassen, während die zweite Kamera optische Bilddaten des Patienten in der Bildaufnahmeregion der Bildgebungsvorrichtung erfasst.

Das Verarbeiten der Prozessdaten umfasst ein Ermitteln einer relativen Position des Patienten zu der Bildgebungsvorrichtung sowie ein Ermitteln einer primären Kamera der Mehrzahl von Kameras in Abhängigkeit der relativen Position des Patienten zu der Bildgebungsvorrichtung. Das Verarbeiten der Prozessdaten erfolgt vorzugsweise mittels einer Bildverarbeitungseinheit der Recheneinheit in Abhängigkeit der erfassten optischen Bilddaten. Es ist aber ebenso vorstellbar, dass das Verarbeiten der Prozessdaten ein Verarbeiten von Prozessdaten zumindest eines weiteren Sensors, wie z. B. eines Abstandssensors und/oder eines Positionsgebers, umfasst. Vorzugsweise erfassen der Abstandssensor und/oder der Positionsgeber eine Position der Patientenlagerungsvorrichtung und/oder des Patiententischs, mittels derer der Patient in der Bildaufnahmeregion der Bildgebungsvorrichtung positioniert wird. Das Ermitteln der primären Kamera kann insbesondere in Abhängigkeit der ermittelten relativen Position des Patienten zu der Bildgebungsvorrichtung erfolgen. Vorzugsweise stellt die primäre Kamera eine Kamera in dem Untersuchungsraum dar, welche zu einem aktuellen Zeitpunkt eine gute oder optimale Sicht auf den Patienten ermöglicht.

Das Bereitstellen der Information bezüglich der Bildgebungsuntersuchung umfasst ein Ausgeben von Bildern der primären Kamera mittels der Ausgabeeinheit. Das Ausgeben von Bildern kann insbesondere auch ein Ausgeben von bewegten Bildern, wie z. B. eines Videos, umfassen.

Durch das Ermitteln der primären Kamera lässt sich auf vorteilhafte Weise eine automatische Auswahl einer Kamera durchführen, welche eine gute oder optimale Sicht auf den Patienten ermöglicht. Ferner können von einer Mehrzahl von Kamers erfasste Bilder automatisch oder manuell durch den Nutzer auf dem Bildbereich der Ausgabeeinheit ausgewählt und/oder angeordnet werden. Dadurch lässt sich eine Qualität der Überwachung des Patienten auf vorteilhafte Weise weiter erhöhen.

In einer weiteren Ausführungsform des erfindungsgemäßen Verfahrens sind die Prozessdaten ausgewählt aus:
- optische Bilddaten aus dem Untersuchungsraum,
- eine Patienteninformation,
- ein medizinischer Parameter des Patienten und/oder
- ein Parameter der Bildgebungsuntersuchung,
wobei das Verarbeiten der Prozessdaten ein Ermitteln einer Priorität der Prozessdaten umfasst und wobei das Bereitstellen der Information bezüglich der Bildgebungsuntersuchung in Abhängigkeit der ermittelten Priorität erfolgt.

Das Ermitteln der Priorität kann insbesondere ein Bestimmen einer Wichtigkeit unterschiedlicher Prozessdaten hinsichtlich eines physiologischen Zustands des Patienten und/oder einer Relevanz der Prozessdaten für die Überwachung des Patienten umfassen. Die Prozessdaten können dabei hinsichtlich der Priorität klassifiziert, sortiert und/oder mit einem vorbestimmten Referenzwert verglichen werden. Vorzugsweise wird das Verarbeiten der Prozessdaten auf solche Prozessdaten beschränkt, welche den vorbestimmten Referenzwert und/oder einen vorbestimmten Rang in einer Prioritätsliste überschreiten.

Durch das Ermitteln der Priorität der Prozessdaten kann das Ermitteln der Information bezüglich der Bildgebungsuntersuchung vorteilhaft auf relevante Prozessdaten eingeschränkt werden. Somit kann ein Rechenaufwand, welcher mit dem erfindungsgemäßen Verfahren verbunden ist, reduziert werden. Ferner lässt sich eine Überlagerung der Sicht des Patienten mit Informationen bezüglich der Bildgebungsuntersuchung auf vorteilhafte Weise vermeiden.

Gemäß einer weiteren Ausführungsform des erfindungsgemäßen Verfahrens umfasst das Verarbeiten der Prozessdaten ein Korrelieren von optischen Bilddaten mit weiteren Prozessdaten. Das Korrelieren von optischen Bilddaten mit weiteren Prozessdaten kann insbesondere ein Korrelieren von Vitaldaten des Patienten mit einer Position des Patienten in dem Untersuchungsraum umfassen. Die Vitaldaten können in dem Bildbereich der Ausgabeeinheit an dem Patienten angeordnet werden. Es ist ebenso vorstellbar, dass das Korrelieren von optischen Bilddaten mit weiteren Prozessdaten ein Ermitteln einer aktuellen Position des Patienten anhand der optischen Bilddaten und ein Korrelieren der aktuellen Position des Patienten mit einer gewünschten Position des Patienten umfasst. Die gewünschte Position des Patienten kann dabei z. B. in Abhängigkeit eines Parameters der Bildgebungsuntersuchung ermittelt werden. Dabei kann insbesondere eine Abweichung zwischen der aktuellen Position des Patienten und der gewünschten Position des Patienten ermittelt werden. Das Korrelieren von optischen Bilddaten mit weiteren Prozessdaten kann weiterhin ein Korrelieren einer bildgebungsrelevanten Komponente mit einer beliebigen Information bezüglich der Bildgebungsuntersuchung umfassen, welche die bildgebungsrelevante Komponente zu einem gegebenen Zeitpunkt betrifft.

Weiterhin umfasst das Bereitstellen der Information bezüglich der Bildgebungsuntersuchung ein Überlagern eines vorbestimmten Bildbereichs der Ausgabeeinheit mit einer grafischen Repräsentation der weiteren Prozessdaten in Abhängigkeit der korrelierten Prozessdaten. Beispielsweise kann die Abweichung in Form einer Markierung und/oder einer graphischen Repräsentation, bereitgestellt und dem Sichtbereich des Nutzers auf den Patienten überlagert werden. Es ist aber ebenso vorstellbar, dass Informationen bezüglich der Bildgebungsuntersuchung basierend auf Vitaldaten des Patienten in Form einer graphischen Repräsentation dem Sichtbereich des Nutzers auf den Patienten überlagert werden. Das Bereitstellen der Information bezüglich der Bildgebungsuntersuchung kann insbesondere eine Anwendung von erweiterter Realität und/oder gemischter Realität umfassen. Hierbei kann eine Wahrnehmung des Nutzers (insbesondere die Überwachung des Patienten) computergestützt um Informationen bezüglich der Bildgebungsuntersuchung erweitert oder angereichert werden.

Durch das Überlagern von erfassten optischen Bilddaten und/oder dem vorbestimmten Bildbereich der Ausgabeeinheit mit einer grafischen Repräsentation von weiteren Prozessdaten kann die Wahrnehmung des Nutzers hinsichtlich der Überwachung des Patienten auf vorteilhafte Weise auf relevante Aspekte der Prozessdaten ausgedehnt werden. Dadurch können bei der Überwachung des Patienten auch solche Informationen berücksichtigt werden, welche nicht von einem Wahrnehmungsbereich des Nutzers umfasst sind.

In einer weiteren Ausführungsform des erfindungsgemäßen Verfahrens umfasst das Erfassen von Prozessdaten ein Erfassen eines Parameters der Bildgebungsuntersuchung, wobei das Verarbeiten der Prozessdaten ein Ermitteln einer Zeitinformation bezüglich eines Vorgangs der Bildgebungsuntersuchung in Abhängigkeit des Parameters der Bildgebungsuntersuchung umfasst. Der Parameter der Bildgebungsuntersuchung kann dabei insbesondere eine Dauer einer Bildgebungssequenz, eines Abschnitts einer Bildgebungssequenz, einer Abfolge von Bildgebungssequenzen, aber auch eine verabreichte Menge eines Kontrastmittels, eine Dauer einer Wirkung des Kontrastmittels, eine verfügbare Menge des Kontrastmittels, eine Zeitdifferenz zu einer nachfolgenden Bildgebungsuntersuchung, eine Überziehung einer gebuchten Dauer für die Bildgebungsuntersuchung oder dergleichen, umfassen. Die in Abhängigkeit des Parameters der Bildgebungsuntersuchung ermittelte Zeitinformation ist vorzugsweise durch eine Information über einen verbleibenden Zeitraum, welcher für eine Durchführung eines Abschnitts bzw. Vorgangs der Bildgebungsuntersuchung zur Verfügung steht, charakterisiert.

Das Bereitstellen der Information bezüglich der Bildgebungsuntersuchung umfasst ein Bereitstellen der ermittelten Zeitinformation in Abhängigkeit des Vorgangs der Bildgebungsuntersuchung. Die ermittelte Zeitinformation wird vorzugsweise mittels der Ausgabeeinheit dem Sichtbereich des Nutzers bei der visuellen Überwachung des Patienten überlagert.

Durch das Bereitstellen der ermittelten Zeitinformation ist der Nutzer imstande, eine Dauer eines Vorgangs der Bildgebungsuntersuchung bei der Überwachung des Patienten zu berücksichtigen. Dies kann den Nutzer vorteilhaft bei einer Entscheidung unterstützen, den Vorgang der Bildgebungsuntersuchung bei einem auffälligen Verhalten des Patienten fortzuführen, zu unterbrechen oder zu wiederholen.

Das erfindungsgemäße Computerprogrammprodukt ist direkt in einen Speicher einer Recheneinheit eines erfindungsgemäßen Überwachungssystems gemäß einer oben beschriebenen Ausführungsform ladbar, mit Programmcode-Mitteln, um ein erfindungsgemäßes Verfahren nach einer oben beschriebenen Ausführungsform auszuführen, wenn das Computerprogrammprodukt in der Recheneinheit des Überwachungssystems ausgeführt wird.

Die Recheneinheit des Überwachungssystems kann in eine Steuereinheit und/oder Recheneinheit der Bildgebungsvorrichtung integriert sein oder als eigenständige Recheneinheit des Überwachungssystems vorliegen. Durch das erfindungsgemäße Computerprogrammprodukt kann das erfindungsgemäße Verfahren schnell, identisch wiederholbar und robust ausgeführt werden. Das Computerprogrammprodukt ist so konfiguriert, dass es mittels der Recheneinheit die erfindungsgemäßen Verfahrensschritte ausführen kann. Die Recheneinheit muss dabei jeweils die Voraussetzungen, wie beispielsweise einen entsprechenden Arbeitsspeicher, eine entsprechende Grafikkarte, eine entsprechende Bildverarbeitungseinheit oder eine entsprechende Logikeinheit aufweisen, so dass die jeweiligen Verfahrensschritte effizient ausgeführt werden können. Das Computerprogrammprodukt ist beispielsweise auf einem computerlesbaren Medium gespeichert oder auf einem Netzwerk, einem Server oder einer Cloud hinterlegt, von wo es in den Prozessor einer lokalen Recheneinheit geladen werden kann.

Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in der Recheneinheit des Überwachungssystems ein erfindungsgemäßes Verfahren durchführen. Beispiele für elektronisch lesbare Datenträger sind eine DVD, ein Magnetband, ein USB-Stick oder beliebige andere Datenspeicher, auf welchen elektronisch lesbare Steuerinformationen, insbesondere Software, gespeichert ist. Wenn diese Steuerinformationen von dem Datenträger gelesen und an die Recheneinheit übertragen werden, können alle erfindungsgemäßen Ausführungsformen des beschriebenen, erfindungsgemäßen Verfahrens durchgeführt werden.

Das erfindungsgemäße Überwachungssystem sowie das erfindungsgemäße Verfahren betreffen insbesondere eine lokale Implementierung bzw. einen lokalen Aufbau in einem Kontrollraum einer Bildgebungsvorrichtung. Im Gegensatz zu einem Fernzugriff bzw. einer Remoteanwendung ist der Nutzer dadurch imstande, bei einem Notfall, welcher beispielsweise mittels der visuellen Überwachung des Patienten festgestellt wird, selbstständig einzugreifen und/oder entsprechende Maßnahmen einzuleiten.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: eine schematische Darstellung einer Ausführungsform einer Bildgebungsvorrichtung mit einem erfindungsgemäßen Überwachungssystem,
- Fig. 2: eine schematische Darstellung einer Ausführungsform eines erfindungsgemäßen Überwachungssystems,
- Fig. 3: eine schematische Darstellung einer Ausführungsform eines erfindungsgemäßen Überwachungssystems,
- Fig. 4: eine schematische Darstellung einer Ausführungsform eines erfindungsgemäßen Überwachungssystems,
- Fig. 5: ein mögliches Ablaufdiagramm einer Ausführungsform eines erfindungsgemäßen Verfahrens.

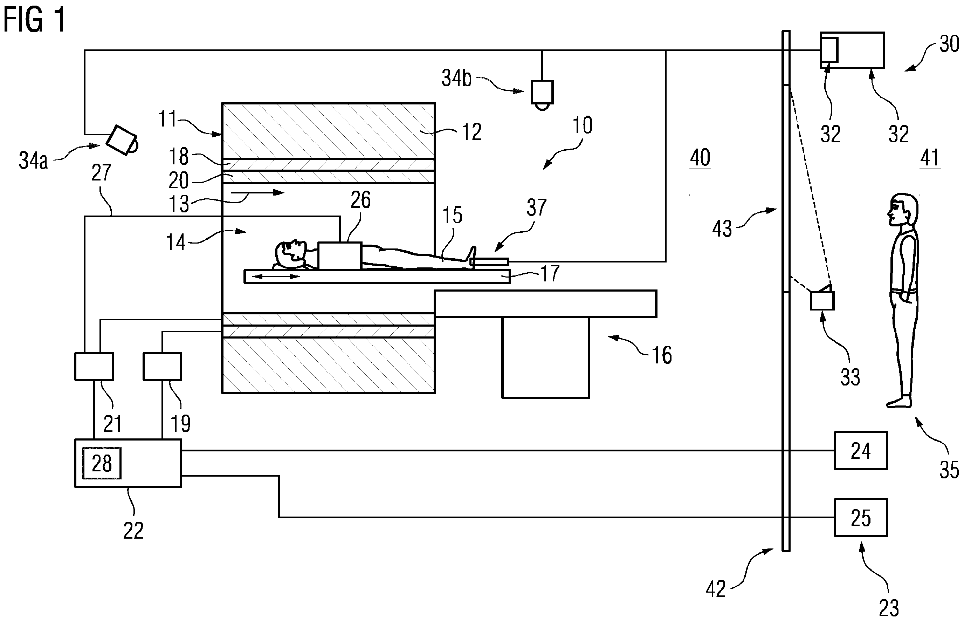

In Fig. 1 ist eine mögliche Ausführungsform der erfindungsgemäßen Bildgebungsvorrichtung 10 dargestellt. Die Bildgebungsvorrichtung 10 ist vorliegend als eine Magnetresonanzvorrichtung 10 ausgestaltet. Es ist aber ebenso vorstellbar, dass die Bildgebungsvorrichtung 10 als ein Computertomograf, eine Röntgenvorrichtung, eine Mammographievorrichtung, ein Positronen-Emissions-Tomograf, ein Einzelphotonen-Emissions-Computertomograf oder dergleichen ausgestaltet ist. Die Magnetresonanzvorrichtung 10 umfasst eine Magneteinheit 11, welche z. B. einen Permanentmagneten, einen Elektromagneten oder einen supraleitenden Hauptmagneten 12 zur Erzeugung eines starken und insbesondere homogenen Hauptmagnetfelds 13 aufweist. Zudem umfasst die Magnetresonanzvorrichtung 10 einen Patientenaufnahmebereich 14 zu einer Aufnahme eines Patienten 15. Der Patientenaufnahmebereich 14 ist im vorliegenden Ausführungsbeispiel zylinderförmig ausgebildet und in einer Umfangsrichtung von der Magneteinheit 11 umgeben. Grundsätzlich sind jedoch auch von diesem Beispiel abweichende Ausbildungen des Patientenaufnahmebereichs 14 vorstellbar.

Der Patient 15 kann mittels einer Patientenlagerungsvorrichtung 16 der Magnetresonanzvorrichtung 10 in dem Patientenaufnahmebereich 14 positioniert werden. Die Patientenlagerungsvorrichtung 16 weist hierfür einen innerhalb des Patientenaufnahmebereichs 14 bewegbar ausgestalteten Patiententisch 17 auf. Die Magneteinheit 11 weist weiterhin eine Gradientenspule 18 zum Erzeugen von magnetischen Gradientenfeldern auf, welche für eine Ortskodierung während einer Bildgebung verwendet wird. Die Gradientenspule 18 wird mittels einer Gradientensteuereinheit 19 der Magnetresonanzvorrichtung 10 angesteuert. Die Magneteinheit 11 kann weiterhin eine Hochfrequenzantenne umfassen, welche im vorliegenden Ausführungsbeispiel als fest in die Magnetresonanzvorrichtung 10 integrierte Körperspule 20 ausgebildet ist. Die Körperspule 20 ist zu einer Anregung von Kernspins ausgelegt, die sich in dem von dem Hauptmagneten 12 erzeugten Hauptmagnetfeld 13 befinden. Die Körperspule 20 wird von einer Hochfrequenzeinheit 21 der Magnetresonanzvorrichtung 10 angesteuert und strahlt hochfrequente Anregungspulse in eine Bildaufnahmeregion ein, die im Wesentlichen von einem Patientenaufnahmebereich 14 der Magnetresonanzvorrichtung 10 gebildet ist. Die Körperspule 20 ist kann weiterhin zum Empfang von Magnetresonanzsignalen ausgebildet sein.

Zu einer Steuerung des Hauptmagneten 12, der Gradientensteuereinheit 19 und zur Steuerung der Hochfrequenzeinheit 21 weist die Magnetresonanzvorrichtung 10 eine Steuereinheit 22 auf. Die Steuereinheit 22 ist dazu ausgebildet eine Durchführung einer Sequenz, wie z. B. einer bildgebenden GRE (gradient echo) Sequenz, einer TSE (turbo spin echo) Sequenz oder einer UTE (ultra-short echo time) Sequenz, zu steuern. Zudem umfasst die Steuereinheit 22 eine Recheneinheit 28 zu einer Auswertung von Magnetresonanzdaten, die während einer Magnetresonanzuntersuchung erfasst werden. Die Recheneinheit 28 der Magnetresonanzvorrichtung 10 kann dazu ausgebildet sein, Rekonstruktionsmethoden einzusetzen, um diagnostische Bilder des Patienten 15 anhand der Magnetresonanzdaten zu rekonstruieren.

Des Weiteren umfasst die Magnetresonanzvorrichtung 10 eine Benutzerschnittstelle 23, welche eine Signalverbindung mit der Steuereinheit 22 aufweist. Steuerinformationen, wie z. B. Bildgebungsparameter, aber auch rekonstruierte Magnetresonanzbilder, können auf einer Anzeigeeinheit 24, beispielsweise auf zumindest einem Monitor, der Benutzerschnittstelle 23 für einen Nutzer angezeigt werden. Weiterhin weist die Benutzerschnittstelle 23 eine Eingabeeinheit 25 auf, mittels der Parameter einer Magnetresonanzuntersuchung von dem Nutzer eingegeben werden können.

Fig. 1 zeigt ferner eine Überwachungssystem 30 mit einer Ausgabeeinheit 33 welche dazu ausgebildet ist, eine Information bezüglich einer Bildgebungsuntersuchung einem Sichtbereich eines Nutzers 35 bei einer visuellen Überwachung des Patienten 15 zu überlagern. Die Ausgabeeinheit 33 ist vorliegend als ein Projektor 33 ausgestaltet, welcher dazu ausgebildet ist, Informationen bezüglich der Bildgebungsuntersuchung 36 in einem Sichtbereich des Nutzers 35 auf eine Oberfläche des Überwachungsfensters 43 zu projizieren. In dem gezeigten Beispiel ist der Projektor 33 zusammen mit dem Nutzer 35 in einem Kontrollraum 41 positioniert, während der Patient 15 und die Magnetresonanzvorrichtung 10 in dem Untersuchungsraum 40 positioniert sind.

Es ist vorstellbar, dass die Benutzerschnittelle 23 mit dem Überwachungssystem 30 gekoppelt ist, sodass eine Steuerung der Ausgabeeinheit 33 mittels der Eingabeeinheit 25 ermöglicht wird. Ferner kann die Schnittstelle 31 des Überwachungssystems 30 eine Signalverbindung mit der Steuereinheit 22 und/oder der Recheneinheit 28 der Magnetresonanzvorrichtung aufweisen, um Prozessdaten, wie z. B. einen Parameter der Bildgebungsuntersuchung, zu empfangen. Ebenso wie die Anzeigeeinheit 24, kann auch die Ausgabeeinheit 33 ausgebildet sein, dem Nutzer 35 Steuerinformationen anzuzeigen und/oder eine Eingabe eines Parameters der Magnetresonanzuntersuchung zu ermöglichen. In dem in Fig. 1 gezeigten Beispiel weist das Überwachungssystem 30 ferner zwei Kameras 34a und 34b auf, welche optische Bilddaten des Patienten 15 erfassen und an die Schnittstelle 31 des Überwachungssystems 30 übermitteln. Die Recheneinheit 32 ist vorzugsweise dazu ausgebildet, den Projektor 33 anzusteuern, die optischen Bilddaten der Kameras 34a und 34b als Bilder und/oder als ein Videostream in einem Sichtbereich des Nutzers 35 bei der visuellen Überwachung des Patienten 15 auf das Überwachungsfenster 43 zu projizieren. Selbstverständlich können Kameras 34 auch in einer von der gezeigten Darstellung abweichenden Konfiguration angeordnet sein. Beispielsweise kann zumindest eine Kamera 34 derart angeordnet sein, dass sie einen Blick des Nutzers 35 auf den Patienten 15 durch ein Überwachungsfenster 43 nachahmt oder imitiert. Es ist ebenso vorstellbar, dass zumindest eine Kamera 34 außerhalb des Untersuchungsraums 40, beispielsweise in einem Patienten-Wartebereich, angeordnet ist. Weiterhin umfasst das erfindungsgemäße Überwachungssystem 30 einen Sensor 37, welcher an dem Patienten 15 positioniert und dazu ausgebildet ist, Vitaldaten des Patienten 15 zu erfassen.

Die Magnetresonanzvorrichtung 10 kann ferner eine lokale Empfangsantenne 26 aufweisen, welche an einer Körperregion des Patienten 15 positioniert ist und Magnetresonanzsignale der Körperregion des Patienten 15 erfasst und an die Recheneinheit 28 der Steuereinheit 22 überträgt. Die lokale Empfangsantenne 26 weist vorzugsweise eine elektrische Anschlussleitung 27 auf, welche eine Signalverbindung mit der Hochfrequenzeinheit 21 und der Steuereinheit 22 bereitstellt. Ebenso wie die Körperspule 20 kann auch die lokale Empfangsantenne 26 zu einer Anregung von Kernspins und zum Empfang von Magnetresonanzsignalen ausgebildet sein. Die lokale Empfangsantenne 26 kann hierfür von der Hochfrequenzeinheit 21 angesteuert werden. In dem vorliegenden Beispiel ist die lokale Empfangsantenne 26 als eine Auflegespule ausgeführt, welche einen Brustkorb des Patienten 15 zumindest teilweise umschließt.

Die dargestellte Magnetresonanzvorrichtung 10 kann selbstverständlich weitere Komponenten umfassen, welche Magnetresonanzvorrichtungen üblicherweise aufweisen. Die Magnetresonanzvorrichtung 10 kann insbesondere dazu ausgebildet ein, eine Magnetresonanzuntersuchung einer Körperregion des Patienten 15 durchzuführen, um diagnostische Bilddaten der Körperregion des Patienten 15 zu erfassen.

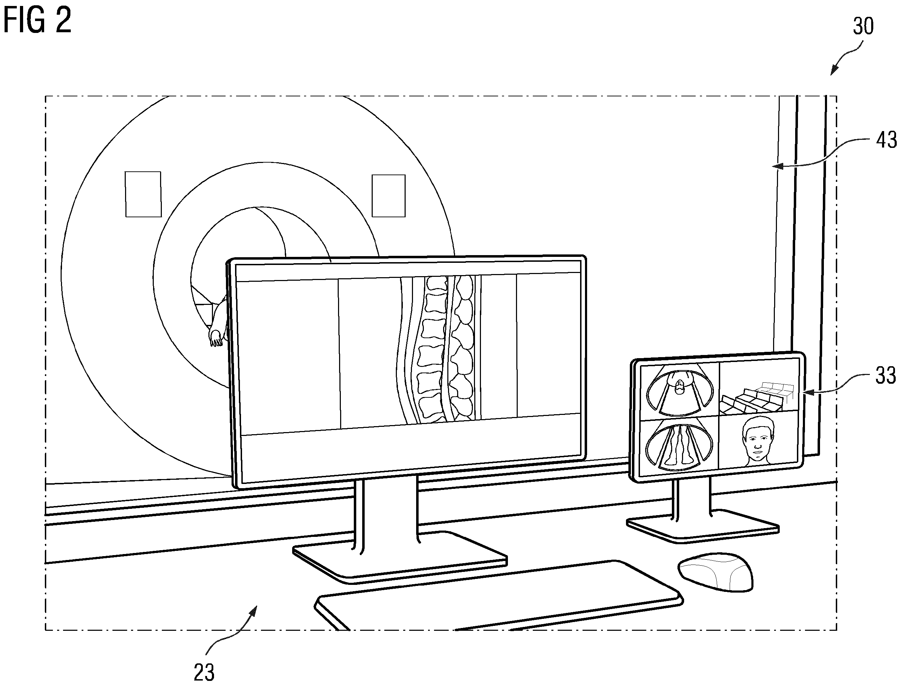

Fig. 2 zeigt eine Ausführungsform des Überwachungssystems 30, bei welcher die Ausgabeeinheit 33 als ein Bildschirm ausgeführt ist. Der Bildschirm 33 kann dabei das Überwachungsfenster 43 ersetzen oder als eine Ergänzung zu dem Überwachungsfenster 43 in dem Kontrollraum 41 installiert sein. Der Bildschirm 33 ist vorliegend in vier Bereiche untergliedert, welche verschiedene Informationen bezüglich der Bildgebungsuntersuchung, insbesondere Videosignale verschiedener Kameras, bereitstellen. Vorliegend ist der Bildschirm 33 derart vor dem Überwachungsfenster 43 positioniert, dass die von dem Bildschirm 33 bereitgestellten Informationen bezüglich der Bildgebungsuntersuchung einer Sicht des Nutzers 35 auf den Patienten 15 (nicht dargestellt) durch das Überwachungsfenster 43 überlagert sind. Es ist weiterhin vorstellbar, dass Bilder des Patienten 15 auf dem Bildschirm 33 durch eine Information bezüglich der Bildgebungsuntersuchung 36 überlagert sind. Bilddaten oder Videosignale einer oder mehrerer Kameras 34 können beispielsweise gemäß eines ONVIF Standards oder eines vergleichbaren Standards auf dem Überwachungssystem 30 implementiert sein.

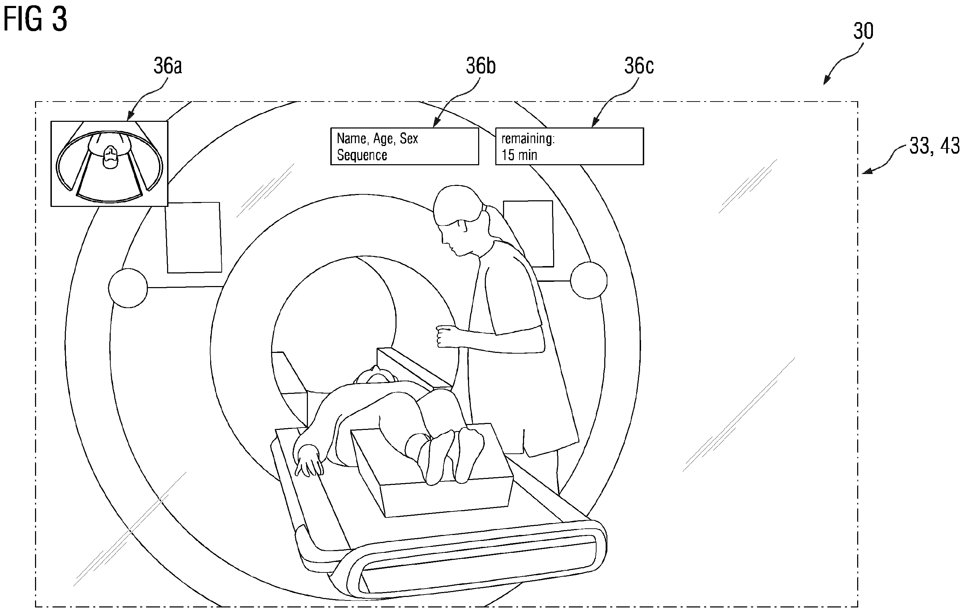

Fig. 3 zeigt eine Ausführungsform des Überwachungssystems 30, bei welcher die Ausgabeeinheit 33 als ein transparenter Bildschirm oder ein Projektor ausgebildet ist. In dem dargestellten Beispiel werden Informationen bezüglich der Bildgebungsuntersuchung 36a, 36b und 36c (36a-c) einem Sichtbereich des Nutzers 35 bei der visuellen Überwachung den Patienten 15 überlagert. Der Nutzer 35 ist somit imstande, direkt auf ausgewählte Informationen bezüglich der Bildgebungsuntersuchung 36a-c in seinem Sichtbereich zuzugreifen und bei der Überwachung des Patienten 15 und/oder der Durchführung der Bildgebungsuntersuchung zu berücksichtigen. Die Informationen bezüglich der Bildgebungsuntersuchung 36a-c beruhen beispielsweise auf besonders relevanten oder priorisierten Prozessdaten, welche von der Recheneinheit 32 ermittelt werden. Im Falle eines transparenten Bildschirms 33 kann das Überwachungsfenster 43 gleichzeitig die Ausgabeeinheit 33 umfassen. Im Falle eine Projektors 33 kann das Überwachungsfenster 43 getrennt von der Ausgabeeinheit 33 vorliegen. Der transparente Bildschirm 33 kann ferner eine Touchscreen-Funktionalität aufweisen, um eine Anpassung eines Parameters der Bildgebungsvorrichtung 10 zu ermöglichen, ohne die visuelle Überwachung des Patienten 15 zu unterbrechen.

In dem gezeigten Beispiel umfasst die Information bezüglich der Bildgebungsuntersuchung 36a ein Videostream des Patienten 15, welcher auf optischen Bilddaten einer Kamera 34 basiert. Die Informationen bezüglich der Bildgebungsuntersuchung 36b und 36c umfassen hingegen Patienteninformationen, wie z. B. einen Namen des Patienten 15, sowie Informationen über eine Art (z. B. "Brain Routine") und einen Fortschritt (z. B. "Remaining 15 min") der Bildgebungsuntersuchung.

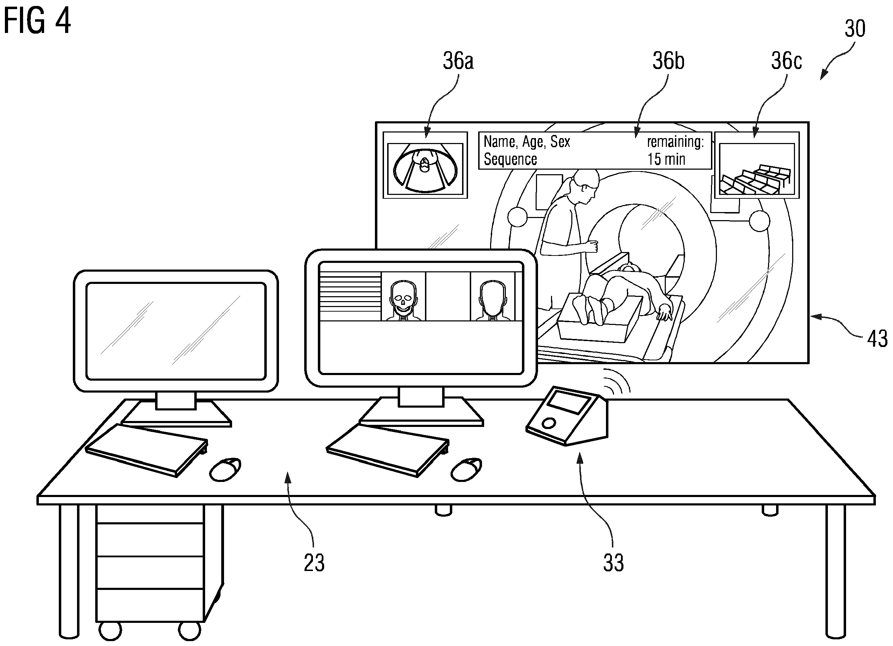

Fig. 4 zeigt eine weitere Ausführungsform des erfindungsgemäßen Überwachungssystems 30, bei welcher die Ausgabeeinheit 33 als ein Projektor oder ein transparenter Bildschirm ausgestaltet ist. Die Informationen bezüglich der Bildgebungsuntersuchung 36a-c können hierbei auch eine Patienteninformation eines nachfolgenden Patienten, aber auch optischen Bilddaten aus einem Patienten-Wartebereich umfassen. Die Recheneinheit 32 kann hierfür eine Signalverbindung mit einer Kamera 34 in dem Patienten-Wartebereich aufweisen. So kann dem Nutzer 35 eine Sicht in den Patienten-Wartebereich bereitgestellt werden. Der Nutzer 35 ist somit imstande einen Zustand des aktuellen Patienten 15 sowie weiteren Patienten im Wartebereich einzuschätzen, während der aktuelle Patient 15 in der Bildgebungsvorrichtung 10 untersucht wird.

Es ist weiterhin vorstellbar, dass eine Funktionalität der Benutzerschnittstelle 23 in das Überwachungssystem 30 integriert ist. Dies kann bedeuten, dass der Nutzer 35 mittels der Ausgabeeinheit 33 diagnostische Bilder der Körperregion des Patienten 15 ansehen und/oder bearbeiten kann. Das Überwachungssystem 30 kann ferner eine Eingabeeinheit aufweisen, welche ein Modifizieren eines Parameters der Bildgebungsuntersuchung, aber auch einer Patienteninformation, ermöglicht.

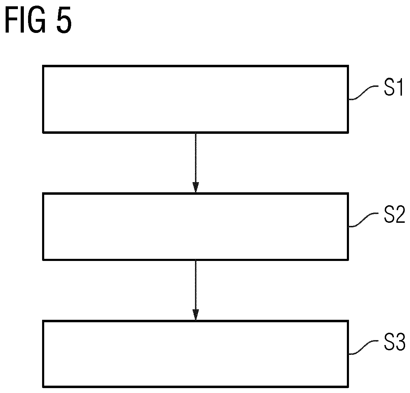

Fig. 5 zeigt ein mögliches Ablaufschema eines erfindungsgemäßen Verfahrens zum Bereitstellen einer Information bezüglich einer Bildgebungsuntersuchung 36 eines Patienten 15 mit einem Überwachungssystem 30 mit einer Schnittstelle 31, einer Recheneinheit 32 und einer Ausgabeeinheit 33.

In einem Schritt S1 werden Prozessdaten mittels der Schnittstelle 31 empfangen. Die Prozessdaten umfassen vorzugsweise optische Bilddaten einer Kamera 34. Es ist aber ebenso vorstellbar, dass die Schnittstelle 31 eine Signalverbindung mit der Steuereinheit 22 und/oder Recheneinheit 28 der Bildgebungsvorrichtung 10 aufweist, um Parameter der Bildgebungsuntersuchung zu empfangen. In einer weiteren Ausführungsform weist die Schnittstelle 31 eine Signalverbindung mit zumindest einem Sensor 37 in dem Untersuchungsraum auf. Der zumindest eine Sensor 37 kann insbesondere dazu ausgebildet sein, physiologische Daten bzw. Vitaldaten des Patienten 15 zu erfassen. Die Prozessdaten können ferner eine Patienteninformation oder einen medizinischen Parameter des Patienten umfassen.

In einem Schritt S2 erfolgt ein Verarbeiten der Prozessdaten mittels der Recheneinheit 32. Das Verarbeiten der Prozessdaten kann insbesondere ein Ermitteln der Information bezüglich der Bildgebungsuntersuchung 36 in Abhängigkeit der Prozessdaten umfassen. In einem Beispiel kann das Verarbeiten der Prozessdaten auch ein Ermitteln einer primären Kamera 34 einer Mehrzahl von Kameras 34 in Abhängigkeit einer relativen Position des Patienten 15 zu der Bildgebungsvorrichtung 10 umfassen.

In einem weiteren Schritt S3 erfolgt ein Bereitstellen der Information bezüglich der Bildgebungsuntersuchung 36 in Abhängigkeit der Prozessdaten mittels der Ausgabeeinheit 33.

In einem weiteren Beispiel umfasst das Verarbeiten der Prozessdaten ein Ermitteln einer Priorität der Prozessdaten. Die dem Nutzer 35 bereitgestellten Informationen bezüglich der Bildgebungsuntersuchung 36 werden dabei in Abhängigkeit der ermittelten Priorität der Prozessdaten ausgewählt und dem Sichtbereich des Nutzers 35 auf den Patienten 15 überlagert.

Das Verarbeiten der Prozessdaten kann ferner ein Korrelieren von optischen Bilddaten mit weiteren Prozessdaten umfassen. Hierbei kann z. B. eine aktuelle Position des Patienten 15 mit einer gewünschten Position des Patienten 15 verglichen und/oder eine Abweichung zwischen der aktuellen Position des Patienten 15 und der gewünschten Position des Patienten 15 ermittelt werden. Die ermittelte Abweichung kann als Information bezüglich der Bildgebungsuntersuchung mittels der Ausgabeeinheit 33 dem Sichtbereich des Nutzers 35 bei der visuellen Überwachung des Patienten 15 überlagert werden.

Das Verarbeiten der Prozessdaten kann ferner ein Ermitteln einer Zeitinformation bezüglich eines Vorgangs der Bildgebungsuntersuchung in Abhängigkeit des Parameters der Bildgebungsuntersuchung umfassen. Die Zeitinformation kann wie in Fig. 3 und Fig. 4 gezeigt mittels der Ausgabeeinheit 33 in dem Sichtbereich des Nutzers 35 bereitgestellt werden.

Selbstverständlich sind die hier beschriebenen Ausführungsformen des erfindungsgemäßen Verfahrens und der erfindungsgemäßen Bildgebungsvorrichtung als beispielhaft zu verstehen. Einzelne Ausführungsformen sind daher um Merkmale anderer Ausführungsformen erweiterbar. Insbesondere ist die Reihenfolge der Verfahrensschritte des erfindungsgemäßen Verfahrens als beispielhaft zu verstehen. Die einzelnen Schritte können auch in einer anderen Reihenfolge durchgeführt werden oder sich zeitlich teilweise oder vollständig überschneiden.

## Patentansprüche

1. Überwachungssystem (30), für eine Überwachung eines Patienten (15) in einem Untersuchungsraum (40) einer Bildgebungsvorrichtung (10), umfassend eine Schnittstelle (31), eine Recheneinheit (32) und eine Ausgabeeinheit (33), wobei die Schnittstelle (31) dazu ausgebildet ist, Prozessdaten bezüglich der Bildgebungsuntersuchung zu empfangen und an die Recheneinheit (32) zu übertragen und wobei die Recheneinheit (32) dazu ausgebildet ist, die Prozessdaten zu verarbeiten und die Ausgabeeinheit (33) anzusteuern, eine Information bezüglich der Bildgebungsuntersuchung (36) in Abhängigkeit der Prozessdaten bereitzustellen, wobei das Überwachungssystem (30) dazu ausgebildet ist, eine visuelle Überwachung des Patienten (15) sowie der Information bezüglich der Bildgebungsuntersuchung (36) mittels der Ausgabeeinheit (33) zu ermöglichen.

2. Überwachungssystem (30) nach Anspruch 1, wobei die Ausgabeeinheit (33) einen Projektor umfasst, wobei der Projektor dazu ausgebildet ist, die Information bezüglich der Bildgebungsuntersuchung (36) auf ein Überwachungsfenster (43) des Untersuchungsraums (40) zu projizieren, wobei das Überwachungsfenster (43) dazu ausgebildet ist, einem Nutzer (35) der Bildgebungsvorrichtung (10) eine Sicht auf den Patienten (15) in dem Untersuchungsraum (40) zu ermöglichen und wobei die Recheneinheit (32) dazu ausgebildet ist, die Ausgabeeinheit (33) anzusteuern, die Information bezüglich der Bildgebungsuntersuchung (36) mittels des Projektors einem Sichtbereich des Nutzers (35) durch das Überwachungsfenster (43) zu überlagern.

3. Überwachungssystem (30) nach Anspruch 1 oder 2, wobei die Ausgabeeinheit (33) einen transparenten Bildschirm aufweist, welcher dazu ausgebildet ist, einem Nutzer (35) der Bildgebungsvorrichtung (10) eine Sicht in den Untersuchungsraum (40) durch den transparenten Bildschirm zu ermöglichen und wobei die Recheneinheit (32) dazu ausgebildet ist, die Ausgabeeinheit (33) anzusteuern, die Information bezüglich der Bildgebungsuntersuchung (36) mittels des transparenten Bildschirms einem Sichtbereich des Nutzers (35) durch den transparenten Bildschirm zu überlagern.

4. Überwachungssystem (30) nach einem der vorhergehenden Ansprüche, wobei das Überwachungssystem (30) ferner eine Kamera (34) aufweist, welche in dem Untersuchungsraum (40) positioniert ist und dazu ausgebildet ist, optische Bilddaten des Patienten (15) in dem Untersuchungsraum (40) zu erfassen und an die Schnittstelle (31) zu übertragen, wobei die Recheneinheit (32) dazu ausgebildet ist, Bilder des Patienten (15) in Abhängigkeit der optischen Bilddaten zu erstellen und mittels der Ausgabeeinheit (33) bereitzustellen.

5. Überwachungssystem (30) nach Anspruch 5, wobei die Ausgabeeinheit (33) einen Bildschirm umfasst und wobei die Recheneinheit (32) dazu ausgebildet ist, Bilder des Patienten (15) in dem Untersuchungsraum (40) mittels des Bildschirms bereitzustellen.

6. Überwachungssystem (30) nach einem der vorhergehenden Ansprüche, ferner aufweisend zumindest einen Sensor (37), welcher in dem Untersuchungsraum (40) der Bildgebungsvorrichtung (10) positioniert ist und dazu ausgebildet ist, Prozessdaten aus dem Untersuchungsraum (40) zu erfassen, wobei die Schnittstelle (31) dazu ausgebildet ist, die Prozessdaten von dem zumindest einen Sensor (37) zu empfangen und an die Recheneinheit (32) zu übertragen, wobei die Information bezüglich der Bildgebungsuntersuchung (36) auf den Prozessdaten des zumindest einen Sensors (37) beruht.

7. Überwachungssystem (30) nach Anspruch 6, wobei der zumindest eine Sensor (37) dazu ausgebildet ist, zumindest eines der folgenden Prozessdaten zu erfassen:
• einen medizinischen Parameter des Patienten (15),
• einen Parameter einer bildgebungsrelevanten Komponente,
• einen Messwert eines Medizingeräts.

8. Überwachungssystem (30) nach einem der vorhergehenden Ansprüche, wobei die Schnittstelle (31) eine Signalverbindung mit der Bildgebungsvorrichtung (10) aufweist und dazu ausgebildet ist, Prozessdaten von der Bildgebungsvorrichtung (10) zu empfangen und and die Recheneinheit (32) zu übertragen, wobei die Prozessdaten einen Parameter der Bildgebungsuntersuchung und/oder eine Patienteninformation betreffen.

9. Überwachungssystem (30) nach einem der vorhergehenden Ansprüche, wobei die Recheneinheit (32) dazu ausgebildet ist, eine Mehrzahl von Informationen bezüglich der Bildgebungsuntersuchung mittels der Ausgabeeinheit (33) bereitzustellen und wobei die Recheneinheit (32) weiterhin dazu ausgebildet ist, eine Art, eine Anordnung und/oder einen Zeitpunkt der bereitgestellten Informationen bezüglich der Bildgebungsuntersuchung (36) in Abhängigkeit der Prozessdaten anzupassen.

10. Verfahren zum Bereitstellen einer Information bezüglich einer Bildgebungsuntersuchung (36) eines Patienten (15) mit einem Überwachungssystem (30) mit einer Schnittstelle (31), einer Recheneinheit (32) und einer Ausgabeeinheit (33) nach einem der vorhergehenden Ansprüche, aufweisend die Schritte:
• Empfangen (S1) von Prozessdaten mittels der Schnittstelle (31),
• Verarbeiten (S2) der Prozessdaten mittels der Recheneinheit (32) und
• Bereitstellen (S3) der Information bezüglich der Bildgebungsuntersuchung (36) in Abhängigkeit der Prozessdaten mittels der Ausgabeeinheit (33).

11. Verfahren nach Anspruch 10, wobei das Empfangen (S1) von Prozessdaten ein Empfangen von optischen Bilddaten einer Mehrzahl von Kameras (34) umfasst, wobei das Verarbeiten (S2) der Prozessdaten ein Ermitteln einer relativen Position des Patienten (15) zu der Bildgebungsvorrichtung (10) sowie ein Ermitteln einer primären Kamera (34) der Mehrzahl von Kameras (34) in Abhängigkeit der relativen Position des Patienten (15) zu der Bildgebungsvorrichtung (10) umfasst, wobei das Bereitstellen (S3) der Information bezüglich der Bildgebungsuntersuchung (36) ein Ausgeben von Bildern der primären Kamera (34) mittels der Ausgabeeinheit (33) umfasst.

12. Verfahren nach einem der Ansprüche 10 oder 11, wobei die Prozessdaten ausgewählt sind aus:
• optische Bilddaten aus dem Untersuchungsraum (40),
• eine Patienteninformation,
• ein medizinischer Parameter des Patienten (15) und/oder
• ein Parameter der Bildgebungsuntersuchung,
wobei das Verarbeiten (S2) der Prozessdaten ein Ermitteln einer Priorität der Prozessdaten umfasst und wobei das Bereitstellen (S3) der Information bezüglich der Bildgebungsuntersuchung (36) in Abhängigkeit der ermittelten Priorität erfolgt.

13. Verfahren nach einem der Ansprüche 10 bis 12, wobei das Verarbeiten (S2) der Prozessdaten ein Korrelieren von optischen Bilddaten mit weiteren Prozessdaten umfasst und wobei das Bereitstellen (S3) der Information bezüglich der Bildgebungsuntersuchung (36) ein Überlagern eines vorbestimmten Bildbereichs der Ausgabeeinheit (33) mit einer grafischen Repräsentation der weiteren Prozessdaten in Abhängigkeit der korrelierten Prozessdaten umfasst.

14. Verfahren nach einem der vorhergehenden Ansprüche 10 bis 13, wobei das Erfassen (S1) von Prozessdaten ein Erfassen eines Parameters der Bildgebungsuntersuchung umfasst, wobei das Verarbeiten (S2) der Prozessdaten ein Ermitteln einer Zeitinformation bezüglich eines Vorgangs der Bildgebungsuntersuchung in Abhängigkeit des Parameters der Bildgebungsuntersuchung umfasst und wobei das Bereitstellen (S3) der Information bezüglich der Bildgebungsuntersuchung (36) ein Bereitstellen der ermittelten Zeitinformation in Abhängigkeit des Vorgangs der Bildgebungsuntersuchung umfasst.

15. Computerprogrammprodukt, welches direkt in einen Speicher einer Recheneinheit (32) eines Überwachungssystems (30) nach einem der Ansprüche 1 bis 9 ladbar ist, mit Programmcode-Mitteln, um ein Verfahren nach einem der Ansprüche 10 bis 14 auszuführen, wenn das Computerprogrammprodukt in der Recheneinheit (32) des Überwachungssystems (30) ausgeführt wird.
